# EUROPÄISCHE PATENTSCHRIFT

(11) **EP 2 417 939 B1**
(45) Veröffentlichungstag und Bekanntmachung des Hinweises auf die Patenterteilung: **29.04.2015**
(21) Anmeldenummer: 10008283.3
(22) Anmeldetag: 09.08.2010
(51) Int. Cl.: A61F 2/44, A61F 2/46

(54) **Wirbelkörperprothese**
Spinal prosthesis
Prothèse de vertèbres

(43) Veröffentlichungstag der Anmeldung: 15.02.2012
(73) Patentinhaber: Röbling, Christian, 79104 Freiburg (DE); Hedayat, Farman, 50931 Köln (DE); Rieger, Claudia, 06108 Halle/Saale (DE)
(72) Erfinder: Röbling, Christian, 79104 Freiburg (DE)
(74) Vertreter: Westphal, Mussgnug & Partner Patentanwälte mbB

(56) Entgegenhaltungen:
- EP-A2- 1 212 992
- DE-U1-202005 009 478
- US-A1- 2005 216 084
- US-A1- 2008 288 073
- US-B1- 6 692 495
- US-B2- 6 984 246

## Beschreibung

Die Erfindung betrifft eine Wirbelkörperprothese gemäß dem Oberbegriff des Patentanspruchs 1.

Bekannt sind Wirbelkörper- und/oder Bandscheibenprothesen mit einer ersten Prothesenplatte und einer zweiten Prothesenplatte zum Abstützen der Prothese gegen die benachbarten Bandscheiben oder Wirbelkörper. Im Nachfolgenden sind von der Bezeichnung "Wirbelkörperprothese" sowohl Prothesen, die lediglich eine Bandscheibe, als auch Prothesen, die lediglich einen Wirbelkörper, sowie Prothesen, die eine beliebige Kombination aus benachbarten Bandscheiben und Wirbelkörpern ersetzen, umfasst.

Bekannt sind Wirbelkörperprothesen mit zwei Prothesenplatten, zwischen welchen ein Faltenbalg angeordnet ist. Derartige Wirbelkörper- und/oder Bandscheibenprothesen zeigen beispielsweise die DE 699 34 024 T2, DE 699 32 946 T2, DE 699 32 448 T2 oder WO 2004/037131 A1.

Dokument US 6692495B offenbart eine Wirbelkörperprothese gemäß des Oberbegriffs von Anspruch 1.

Die Aufgabe der Erfindung besteht darin, eine derartige Wirbelkörperprothese dahingehend zu verbessern, dass sie flexibler einsetzbar ist.

Die Aufgabe wird erfindungsgemäß gelöst durch eine Wirbelkörperprothese mit den Merkmalen des Patentanspruchs 1.

Vorteilhafte Ausgestaltungen und Weiterbildungen der Erfindung sind in den Unteransprüchen angegeben.

Die erfindungsgemäße Wirbelkörperprothese mit einer ersten Prothesenplatte und einer zweiten Prothesenplatte zum Abstützen der Wirbelkörperprothese gegen die benachbarten Bandscheiben oder Wirbelkörper zeichnet sich dadurch aus, dass zwischen der ersten Prothesenplatte und der zweiten Prothesenplatte ein Faltenbalg angeordnet ist und der Faltenbalg einen Innenraum aufweist, welche durch eine Trennwand in wenigstens zwei Kammern getrennt ist. Der Faltenbalg eröffnet die Möglichkeit, einerseits die Prothese mit zusammengefaltetem Faltenbalg in den Zwischenwirbelraum einzuführen und erst in situ die Höhe zu verändern, andererseits die Wirbelkörperprothese mit beliebig gegeneinander gekippten Prothesenplatten in den Zwischenwirbelraum einzusetzen, sodass sich die Wirbelkörperprothese optimal an den Zwischenwirbelraum anpassen kann. Der Faltenbalg hat dabei den Vorteil, dass er sich lediglich in eine Richtung aufstellt, dabei jedoch eine beliebige Verkippung der beiden Prothesenplatten gegeneinander ermöglicht, gegebenenfalls auch mit einem radialen Versatz der Prothesenplatten gegeneinander. Durch die Trennwand und die Aufteilung des Innenraums in wenigstens zwei Kammern wird es ermöglicht, die beiden Kammern unterschiedlich hoch mit Flüssigkeit oder einer pastösen Masse, beispielsweise mit Knochenzement, zu füllen, um auf diese Art und Weise die Wirbelkörperprothese besonders flexibel einsetzen zu können und an die anatomischen Gegebenheiten des Zwischenwirbelraumes anzupassen.

Vorzugsweise verläuft dazu die wenigstens eine Trennwand in Längsrichtung des Faltenbalgs, um auf unterschiedliche Abstände in Längsrichtung der Wirbelsäule zwischen den benachbarten Wirbelkörpern oder Bandscheiben eingehen zu können.

Eine besonders gute Flexibilität der Wirbelkörperprothese wird erreicht, wenn der Faltenbalg vier Kammern aufweist.

Um die einzelnen Kammern auf einfache Art und Weise unabhängig voneinander mit Flüssigkeit oder einer pastösen Masse, beispielsweise Knochenzement, füllen zu können, sind vorzugsweise für jede Kammer an der ersten Prothesenplatte und/oder der zweiten Prothesenplatte insgesamt wenigstens ein, vorzugsweise zwei, Ventile angeordnet. Ein Ventil bietet die Möglichkeit, nach Bedarf eine Flüssigkeit oder Knochenzement in die Wirbelkörperprothese einfüllen zu können, auch nachdem die Wirbelkörperprothese bereits in den Zwischenwirbelraum eingebracht wurde, und dort in situ die Flüssigkeit oder den Knochenzement derart einzufüllen, bis die optimale Füllung der Wirbelkörperprothese erreicht ist.

Bei Verwendung von zwei Ventilen ist es dabei sowohl möglich, dass beide Ventile an einer der beiden Prothesenplatten angeordnet sind, als auch, dass jede der beiden Prothesenplatten jeweils wenigstens ein Ventil aufweist. Zwei Ventile bieten den Vorteil, dass zunächst nach Einbringen der Wirbelköperprothese in den Zwischenwirbelraum über ein Ventil eine Flüssigkeit, insbesondere ein Röntgenkontrastmittel, beispielsweise Bariumsulfat, eingebracht werden kann und anschließend die Wirbelkörperprothese optimal im Zwischenwirbelraum positioniert werden kann. Erst wenn eine gewünschte Füllung der Wirbelkörperprothese erreicht ist und eine günstige Position der Wirbelkörperprothese im Zwischenwirbelraum festgestellt wurde, wird über eines der Ventile Knochenzement nachgespritzt, wobei über das andere Ventil das Röntgenkontrastmittel wieder aus der Wirbelkörperprothese austreten kann, sodass nach und nach die Wirbelkörperprothese mit dem Knochenzement gefüllt wird, während die Wirbelkörperprothese in der gewünschten günstigen Position verbleibt. Sobald an den Auslassventilen Knochenzement sichtbar wird, ist die Wirbelkörperprothese vollständig mit Knochenzement gefüllt, welcher dann in der Wirbelkörperprothese aushärten kann, ohne dass die Wirbelkörperprothese nochmals neu positioniert werden muss. Auf diese Art und Weise kann die Wirbelkörperprothese einfach in einer günstigen Position stabilisiert werden.

Vorzugsweise sind die Ventile als Einwegventile ausgebildet, wobei insbesondere über ein Ventil lediglich Flüssigkeit oder eine pastöse Masse wie Knochenzement in den Wirbelkörper eingefüllt werden kann und über das andere Ventil lediglich das entsprechende Fluid dem der Wirbelkörperprothese austreten kann.

Vorzugsweise ist der Faltenbalg gedichtet an den Prothesenplatten fixiert, um ein Austreten des eingefüllten Materials zu vermeiden.

Eine vorteilhafte Ausführungsform der Erfindung sieht vor, dass der Faltenbalg aus einem nicht dehnfähigen Material gefertigt ist, um unerwünschtes Deformieren des Faltenbalgs zu vermeiden.

Besonders bevorzugt ist der Faltenbalg gewebeverstärkt ausgebildet, um eine ausreichende Stabilität des Faltenbalgs und der Wirbelkörperprothese zu gewährleisten.

Gemäß einer besonders bevorzugten Ausführungsform der Erfindung sind im dem Faltenbalg wenigstens ein, vorzugsweise mehrere Drähte zur Verstärkung angeordnet. Dabei sind vorteilhafterweise die Drähte zu Ringen gebogen in dem Faltenbalg angeordnet, um eine Stabilität des Faltenbalgs insbesondere in radialer Richtung zu gewährleisten. Gemäß einer vorteilhaften Ausführungsform der Erfindung sind die Drähte als Nitinoldrähte ausgebildet, welche eine besonders gute Formgebung ermöglichen.

Der Faltenbalg ist vorzugsweise aus Kunststoff, insbesondere einem thermoplastischen Kunststoff, insbesondere PET (Polyethylenterephthalat) gefertigt, um die Biokompatibilität zu gewährleisten und andererseits einen möglichst stabilen Faltenbalg bereitzustellen.

Die Erfindung wird anhand der nachfolgenden Figuren ausführlich erläutert.

Es zeigt:
- Fig. 1: eine Seitenansicht eines Ausführungsbeispiels einer Wirbelkörperprothese,
- Fig. 2: eine Explosionsdarstellung der Wirbelkörperprothese gemäß Figur 1,
- Fig. 3: eine perspektivische Ansicht einer Prothesenplatte der Wirbelkörperprothese gemäß Figur 2,
- Fig. 4: eine Draufsicht auf die Prothesenplatte gemäß Figur 3,
- Fig. 5: ein Instrument zum Einsetzen der Wirbelkörperprothese gemäß Figur 1,
- Fig. 6: eine perspektivische Ansicht der Wirbelkörperprothese gemäß Figur 1,
- Fig. 7: die Wirbelkörperprothese gemäß Figur 1 im zwischen zwei Wirbelkörper eingesetzten Zustand,

- Fig. 8: eine perspektivische Ansicht der Wirbelkörperprothese gemäß Figur 1 mit unterschiedlich hoch gefüllten Kammern,
- Fig. 9: eine perspektivische Ansicht der Wirbelkörperprothese gemäß Figur 1 mit etwa gleich hoch gefüllten Kammern und
- Fig. 10: eine Draufsicht auf eine Prothesenplatte der Wirbelkörperprothese gemäß Figur 1.

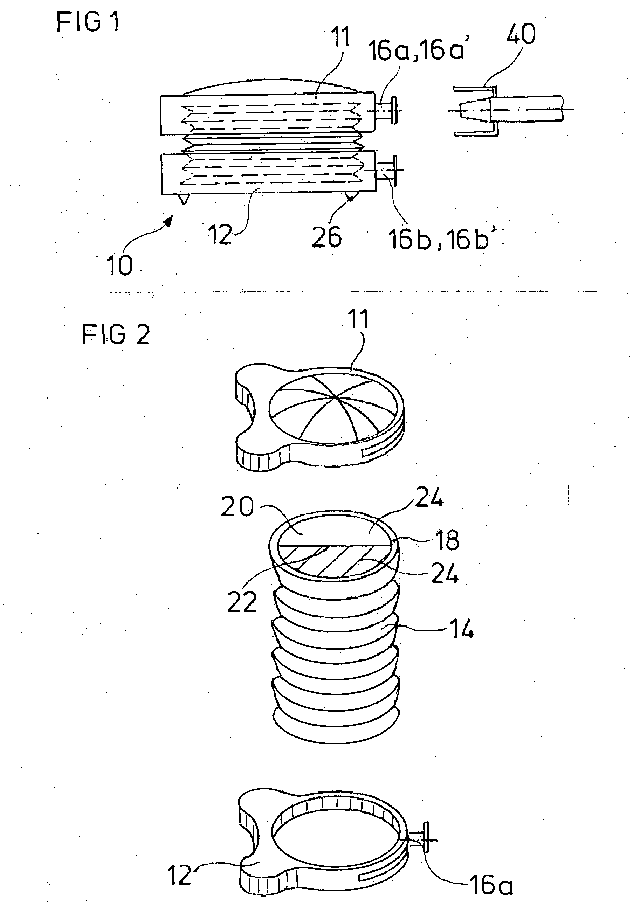

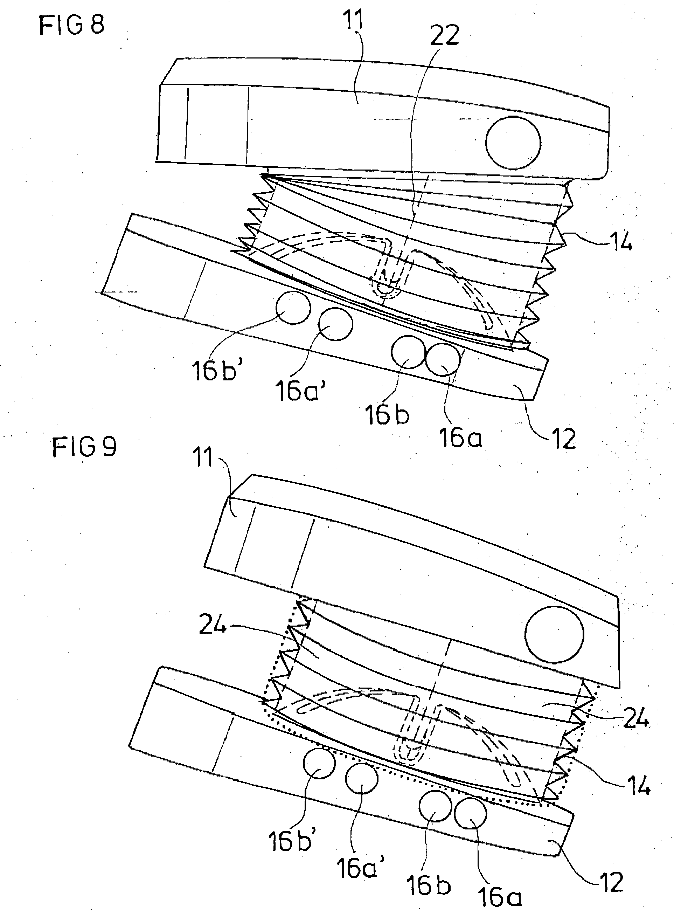

Die Figuren 1 bis 4 und 6 bis 10 zeigen verschiedene Ansichten eines ersten Ausführungsbeispiels einer Wirbelkörperprothese 10 mit einer ersten Prothesenplatte 11 und einer zweiten Prothesenplatte 12. Zwischen den beiden Prothesenplatten 11, 12 ist ein Faltenbalg 14 angeordnet. Der Faltenbalg 14 ist mit seinem oberen Rand gedichtet an der ersten Prothesenplatte 11 befestigt, während er mit seinem unteren Rand gedichtet in der zweiten Prothesenplatte 12 befestigt ist. Der Faltenbalg 14 ist aus einem nicht dehnfähigen Material gefertigt, insbesondere aus Kunststoff. Vorzugsweise wird ein thermoplastischer Kunststoff, beispielsweise PET (Polyethylenterephthalat) zur Fertigung des Faltenbalgs 14 verwendet. Der Faltenbalg 14 ist insbesondere gewebeverstärkt ausgebildet. Der Faltenbalg 14 weist Drähte, insbesondere Nitinoldrähte, zur Verstärkung auf. Die Drähte sind insbesondere zu Ringen gebogen in dem Faltenbalg 14 angeordnet.

In dem Innenraum 20 des Faltenbalgs 14 ist eine Trennwand 22 angeordnet ist, welche in Längsrichtung des Faltenbalgs 14 verlaufend angeordnet ist und den Innenraum 20 des Faltenbalgs 14 in zwei Kammern 24 trennt. Insbesondere ist die Trennwand 22 derart angeordnet, dass die beiden Kammern 24 gegeneinander vollständig getrennt sind, sodass keine Flüssigkeit oder pastöse Masse aus der einen Kammer in die andere Kammer übertreten kann. Für jede der beiden Kammern 24 sind zwei Ventile 16a, 16b bzw. 16a', 16b' vorgesehen, sodass jede der beiden Kammern 24 wie nachfolgend beschrieben unabhängig voneinander mit Flüssigkeit bzw. Knochenzement gefüllt werden kann, um auf diese Art und Weise die Wirbelkörperprothese flexibler einsetzen zu können. Es ist selbstverständlich auch möglich, mehr als zwei Kammern 24, beispielsweise drei oder vier Kammern, vorzusehen, wobei vorzugsweise für jede der Kammern 24 zwei Ventile vorgesehen werden.

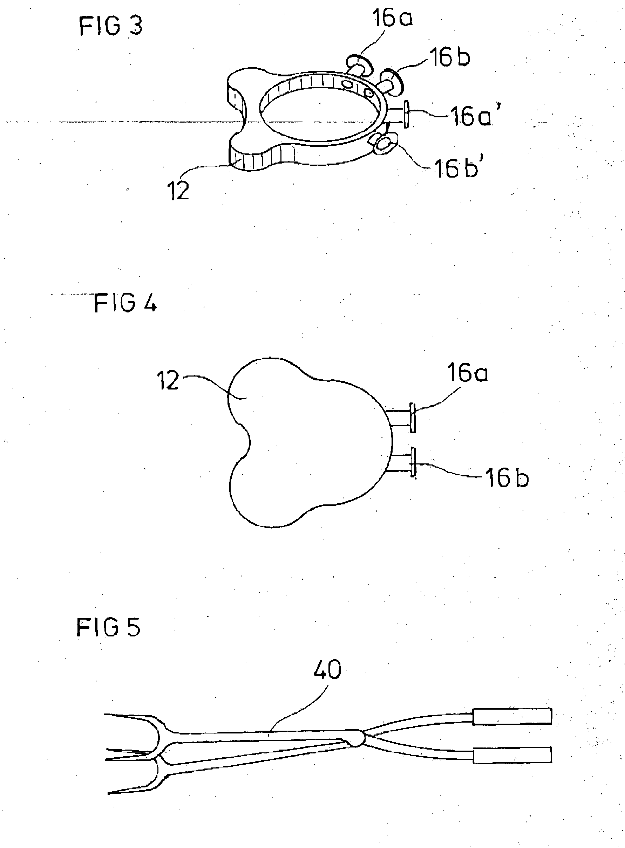

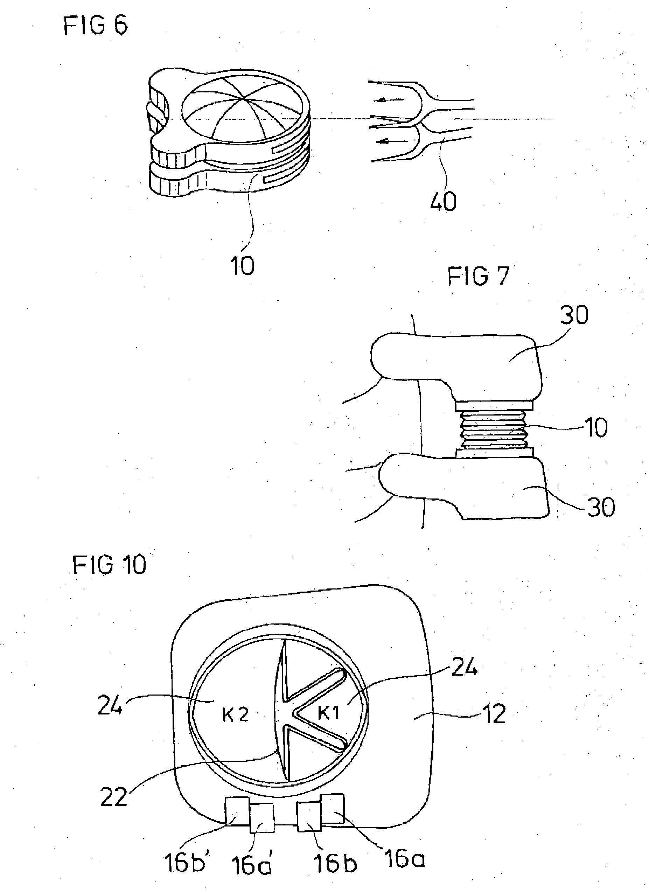

Die vier Ventile 16a, 16b, 16a', 16b' können, wie in Figur 3 dargestellt, an einer der beiden Prothesenplatten 12 angeordnet werden. Alternativ sind, wie in Figur 1 dargestellt, an jeder der beiden Prothesenplatten 11, 12 jeweils zwei Ventile 16a, 16a', 16b, 16b' angeordnet. Die Ventile 16a, 16b, 16a', 16b' sind vorzugsweise als Einwegventile ausgebildet, wobei bei Verwendung von Einwegventilen eines der beiden Ventile 16a, 16a' das Einbringen von Flüssigkeit oder einer pastösen Masse in den Innenraum 20 des Faltenbalgs 14 ermöglicht, während das andere der beiden Ventile 16b, 16b' das Austreten von Flüssigkeit oder einer pastösen Masse aus dem Innenraum 20 des Faltenbalgs 14 ermöglicht. Dadurch wird eine besonders günstige Operationsmethode ermöglicht, bei welcher zunächst die Wirbelkörperprothese im zusammengeklappten Zustand, wie in Figur 1 gezeigt, mit Hilfe eines Einsetzinstruments 40, das in Figur 5 näher dargestellt ist, in den Zwischenwirbelraum eingesetzt wird. Anschließend wird ein Röntgenkontrastmittel, beispielsweise Bariumsulfat, über eines der Ventile 16a, 16a' in den Innenraum 20 des Faltenbalgs 14 eingebracht, bis die gewünschte Füllung des Innenraums 20 des Faltenbalgs 14 und die gewünschte Position der Wirbelkörperprothese 10 innerhalb des Zwischenwirbelraums erreicht ist. Anschließend wird über das Ventil 16a, 16a' Knochenzement nachgespritzt, wobei das Röntgenkontrastmittel über das andere Ventil 16b, 16b' aus der Wirbelkörperprothese 10 wieder austreten kann, bis schließlich die Wirbelkörperprothese 10 mit der gewünschten Menge an Knochenzement in der zuvor ermittelten Position der Wirbelkörperprothese 10 gefüllt ist. Die schematische Positionierung der Wirbelkörperprothese 10 zwischen zwei benachbarten Wirbelkörpern 30 zeigt Figur 7. In diesem Fall ersetzt die Wirbelkörperprothese 10 sowohl einen Wirbelkörper als auch die beiden benachbarten Bandscheiben. Die Wirbelkörperprothese 10 kann jedoch auch lediglich einen Wirbelkörper, eine Bandscheibe oder eine beliebige Kombination aus benachbarten Wirbelkörpern und Bandscheiben ersetzen.

Die Wirbelkörperprothese 10 kann mit Hilfe des Einsetzinstruments 40, das in Figur 5 dargestellt ist, eingebracht werden. Das Einsetzinstrument ist nach Art einer Zange ausgebildet, wobei die Arbeitsenden der Zange jeweils an einer der Prothesenplatten 11, 12 angreifen, beispielsweise diese klemmend am Außenrand umfassen. Zum Einsetzen werden die Prothesenplatten 11, 12 möglichst zusammengedrückt, der Faltenbalg 14 ist dabei zusammengefaltet. Erst nach Einsetzen in den Zwischenwirbelraum wird die Zange leicht aufgespreizt, um die Wirbelkörperprothese 10 an den Zwischenwirbelraum anzupassen.

### Bezugszeichenliste

- 10: Wirbelkörperprothese
- 11: Prothesenplatte
- 12: Prothesenplatte
- 14: Faltenbalg
- 16a: Ventil
- 16a': Ventil
- 16b: Ventil
- 16b': Ventil
- 18: Draht
- 20: Innenraum
- 22: Trennwand
- 24: Kammer
- 30: Wirbelkörper
- 40: Einsetzinstrument

## Patentansprüche

1. Wirbelkörperprothese (10) mit einer ersten Prothesenplatte (11) und einer zweiten Prothesenplatte (12) zum Abstützen der Wirbelkörperprothese regen die benachbarten Bandscheiben oder Wirbelkörper (30), wobei zwischen der ersten Prothesenplatte (11) und der zweiten Prothesenplatte (12) ein Faltenbalg (14) angeordnet ist und der Faltenbalg (14) einen Innenraum (20) aufweist, welcher durch eine Trennwand (22) in wenigstens zwei Kammern (24) getrennt ist,
**dadurch gekennzeichnet, dass** für jede Klammer (24) an der ersten Prothesenplatte (11) und/oder der zweiten Prothesenplatte (12) insgesamt zwei Ventile (16a, 16a', 16b, 16b') angeordnet sind.

2. Wirbelkörperprothese nach Anspruch 1,
**dadurch gekennzeichnet, dass** die wenigstens eine Trennwand (22) in Längsrichtung des Faltenbalgs (14) verlaufend angeordnet ist.

3. Wirbelkörperprothese nach einem der vorhergehenden Ansprüche,
**dadurch gekennzeichnet, dass** der Faltenbalg (14) vier Kammern (24) aufweist.

4. Wirbelkörperprothese nach einem der Ansprüche 1-3,
**dadurch gekennzeichnet, dass** die Ventile (16a, 16a', 16b, 16b') als Einwegventil ausgebindet sind.

5. Wirbelkörperprothese nach einem der vorhergehenden Ansprüche,
**dadurch gekennzeichnet, dass** der Faltenbalg (14) gedichtet an den Prothesenplatten (11 12) fixiert ist.

6. Wirbelkörperprothese nach einem der vorhergehenden Ansprüche;
**dadurch gekennzeichnet, dass** der Faltenbalg (14) aus einen nicht-dehnfähiaen Material gefertigt ist.

7. Wirbelkörperprothese nach einem der vorhergehenden Ansprüche,
**dadurch gekennzeichnet, dass** der Faltenbalg (14) gewebeverstärkt ausgebildet ist.

8. Wirbelkörperprothese nach einem der vorhergehenden Ansprüche,
**dadurch gekennzeichnet, dass** in dem Faltenbalg (14) wenigstens ein Draht (18) zur Verstärkung angeordnet ist.

9. Wirbelkörperprothese nach einem der vorhergehenden Ansprüche,
**dadurch gekennzeichnet, dass** der Draht (18) als Nitinoldraht ausgebildet ist.

10. Wirbelkörperprothese nach einem der vorhergehenden Ansprüche,
**dadurch gekennzeichnet, dass** der Draht (18) zu einem Ring gebogen in dem Faltenbalg (14) angeordnet ist.

11. Wirbelkörperprothese nach einem der vorhergehenden Ansprüche,
**dadurch gekenntzeichnet,** dass der Faltenbalg (14) aus Kunststoff, insbesondere einem thermoplastischen Kunststoff, insbesondere aus PET (Polyethylentexephthalat) gefertigt ist.

## Claims

1. Vertebral body prosthesis (10) with a first prosthesis plate (11) and a second prosthesis plate (12) for supporting the vertebral body prosthesis against the neighbouring intervertebral disks or vertebral bodies (30), wherein a bellows (14) is arranged between the first prosthesis plate (11) and the second prosthesis plate (12), the bellows (14) having an inner space (20), which is separated by a separation wall (22) in at least two chambers (24), **characterized in that** in total two valves (16a, 16a', 16b, 16b') for each chamber (24) are arranged on the first prosthesis plate (11) and/or the second prosthesis plate (12).

2. Vertebral body prosthesis according to claim 1, **characterized in that** the at least one separation wall (22) is arranged running in the longitudinal direction of the bellows (14).

3. Vertebral body prosthesis according to any one of the above claims, **characterized in that** the bellows (14) comprise four chambers (24).

4. Vertebral body prosthesis according to any one of claims 1 - 3, **characterized in that** valves (16a, 16a', 16b, 16b') are formed as one-way valves.

5. Vertebral body prosthesis according to any one of the above claims, **characterized in that** the bellows (14) is secured sealed on the prosthesis plates (11, 12).

6. Vertebral body prosthesis according to any one of the above claims, **characterized in that** the bellows (14) is made from non-stretchable material.

7. Vertebral body prosthesis according to any one of the above claims, **characterized in that** the bellows (14) is formed reinforced with fabric.

8. Vertebral body prosthesis according to any one of the above claims, **characterized in that** at least one wire (18) is arranged in the bellows (14) for reinforcement.

9. Vertebral body prosthesis according to any one of the above claims, characterized that the wire (18) is formed of nitinol wire.

10. Vertebral body prosthesis according to any one of the above claims, **characterized in that** the wire (18) is arranged bent to form a ring in the bellows (14).

11. Vertebral body prosthesis according to any one of the above claims, **characterized in that** the bellows (14) is made from plastic, particularly a thermoplastic, more particularly PET (polyethylene terephthalate).

## Revendications

1. Prothèse de corps vertébral (10) comprenant une première plaque de prothèse (11) et une seconde plaque de prothèse (12) pour permettre à la prothèse de corps vertébral de s'appuyer contre les disques intervertébraux ou les corps vertébraux (30) voisins, un soufflet (14) étant positionné entre la première plaque de prothèse (11) et la seconde plaque de prothèse (12) et ce soufflet (14) comportant un volume interne (20) qui est subdivisé par une paroi de séparation (22) en au moins deux chambres (24),
**caractérisée en ce que**
pour chacune des chambres (24) deux soupapes (16a, 16a', 16b, 16b') sont globalement montées sur la première plaque de prothèse (11) et/ou sur la seconde plaque de prothèse (12).

2. Prothèse de corps vertébral conforme à la revendication 1,
**caractérisée en ce que**
la paroi de séparation (22) s'étend en continu dans la direction longitudinale du soufflet (14).

3. Prothèse de corps vertébral conforme à l'une des revendications précédentes,
**caractérisée en ce que**
le soufflet (14) comporte quatre chambres (24).

4. Prothèse de corps vertébral conforme à l'une des revendications 1 à 3,
**caractérisée en ce que**
les soupapes (16a, 16a', 16b, 16b') sont réalisées sous la forme de soupapes unidirectionnelles.

5. Prothèse de corps vertébral conforme à l'une des revendications précédentes,
**caractérisée en ce que**
le soufflet (14) est fixé de manière étanche sur les plaques de prothèse (11, 12).

6. Prothèse de corps vertébral conforme à l'une des revendications précédentes,
**caractérisée en ce que**
le soufflet (14) est réalisé en un matériau non extensible.

7. Prothèse de corps vertébral conforme à l'une des revendications précédentes,
**caractérisée en ce que**
le soufflet (14) est renforcé par un tissu.

8. Prothèse de corps vertébral conforme à l'une des revendications précédentes,
**caractérisée en ce qu'**
au moins un fil de renfort (18) est monté dans le soufflet (14).

9. Prothèse de corps vertébral conforme à l'une des revendications précédentes,
**caractérisée en ce que**
le fil (18) est réalisé sous la forme d'un fil de Nitinol.

10. Prothèse de corps vertébral conforme à l'une des revendications précédentes,
**caractérisée en ce que**
le fil (18) est recourbé en un anneau dans le soufflet (14).

11. Prothèse de corps vertébral conforme à l'une des revendications précédentes,
**caractérisée en ce que**
le soufflet (14) est réalisé en un matériau synthétique, en particulier en un matériau synthétique thermoplastique, en particulier en PET (Polyéthylène téréphtalate).
